# EUROPEAN PATENT APPLICATION

(11) **EP 4 736 788 A1**
(43) Date of publication of application: **06.05.2026**
(21) Application number: 24315511.6
(22) Date of filing: 05.11.2024
(51) Int. Cl.: A61B 17/22

(54) **CATHETER DEVICE AND SYSTEM FOR REMOVING A THROMBUS FROM A VESSEL**

(71) Applicant: Artedrone, 75013 Paris (FR)
(72) Inventor: The designation of the inventor has not yet been filed
(74) Representative: Hepp Wenger Ryffel AG

(57) **Abstract**

The invention relates to a catheter device (1) for use in removing or assisting in removing a thrombus (T) from a vessel (V). The catheter device (1) comprises a first shaft (2), a distal anchor (3), and an activator (4). The distal anchor (3) is attached to a distal end portion (5) of the first shaft (2), and the activator (4) is configured to expand the distal anchor(3). The distal portion (5) of the first shaft (2) is adapted to penetrate the thrombus (T) and/or pass next to the thrombus (T) such as to exert a force on the thrombus (T) from a distal side, via the distal anchor (3), when the first shaft (2) is pulled.

## Description

The present invention relates to a catheter device, systems and methods according to the preamble of the independent claims.

Devices for removing blot clots are known in the art.

For example, US 2019/374128 A1 discloses a device which can detect and retrieve a blood clot by advancing a catheter with a clot sensing element through a patient vascular.

WO 2017/161204 A1 discloses devices, systems and methods for removal of a thrombus or embolus from a vascular channel of the body. The device comprises an expandable scaffold which is inserted into the vascular channel and positioned at the thrombus or embolus in a collapsed configuration.

Devices in known in the art present several disadvantages. For example, these devices may exert a force which might break a blood clot. As a result, clot fragments may be washed away and carried downstream, leading to further vessel blockages.

Thus, the object of the present invention is to overcome the drawbacks of the prior art, in particular to provide a medical device for improved blood clot removal.

This and other objects are achieved by the catheter device, systems, and the methods according to the characterizing portion of the independent claims of the invention.

The invention is directed to a catheter device for use in moving or removing, or assisting in moving or removing, a thrombus within (or from) a vessel. The catheter device comprises a first shaft,a distal anchor, and an activator. The distal anchor is attached to a distal end portion of the first shaft. The activator is configured to expand the distal anchor. The distal portion of the first shaft is adapted to engage a thrombus on a distal side of the thrombus, for example by penetrating the thrombus and/or to passing next to the thrombus. Preferably, the distal portion of the first shaft may extend from a first side of the thrombus to a second side of the thrombus, such as to exert a force on the thrombus from a distal side, via the distal anchor, when the first shaft is pulled.

The catheter device may obviate the need for guidewires or other assisting devices when navigating and placing the catheter device at a target site.

The catheter device may also ensure that that other device which attach to the thrombus from a proximal side (such as aspiration catheters, suctions cups, and/or others) remain in contact with the thrombus, thereby making the treatment safer. The distal anchor may be controlled to pull the thrombus back if a contact to the other device at a proximal side is insufficient. Monitoring of the contact may be performed by any means known in the art, e.g., imaging, sensors, measuring of a variation of a distance between the devices (e.g. catheter device and aspiration catheter/suction cup/etc.) and such.

Furthermore, the catheter device may avoid fragmentation of the blood clot. The device according to the invention is also adapted to retrieve a thrombus without relying on fragmentation by other means (e.g. lytic agents).

The distal portion of the first shaft may have a stiffness sufficient to push and pierce through a thrombus, even if some bending may occur. Stiffness may be adapted by material choice, dimensioning, and/or geometry of the distal portion.

Distal may be understood, in the context of the present disclosure, a general direction away from a user. A distal portion may thus be further away from another portion (e.g. a proximal portion, or a medical device such as a suction or aspiration device) in a distal direction, but it does not need to be the distal-most portion of the device. Similarly, a distal anchor is not necessarily the distal-most portion of the catheter device or medical device.

The distal anchor may comprise or consist of hooks and/or barbs. For example, hooks and/or barbs may be arranged on a central shaft to engage the thrombus. Additionally or alternatively, hooks or barbs may be arranged on an expandable element, e.g. an arm.

When the distal portion is configured to pass next to the thrombus, the distal portion may be adapted to take the path with less resistance, and thereby may bend to pass between the blood clot and the wall. The rigidity of the distal portion may, in particular, be adapted to achieve this result. Additionally, a magnetic element may be arranged distally such that a magnetic field may be utilized to ensure the progression of the distal portion between the blood clot and the vessel wall.

The catheter device may be delivered over the artery wall. The catheter device may have an opening in a side wall of the main channel through which the distal portion may exit, thus ensuring that the catheter device is not delivered in the center of the clot.

A hook may be arranged on an external surface of the catheter device, e.g. on a suction cup. The hook may help to guide the catheter device between the clot and the artery wall. The hook may have an inner diameter between 0.2 mm and 0.7 mm.

Magnetic attraction may be used guide and orient a magnetic distal part of the catheter device toward the inner surface of the artery.

The catheter device may thus be used to navigate up to a thrombus and to deploy the distal anchor after passing by or through the thrombus, i.e. to place the distal anchor distally behind the thrombus. By expanding the distal anchor, a support may be formed which may push against a distal surface of the thrombus. When a force is exerted on the distal anchor, e.g. via the first shaft, the thrombus may be removed or such a removal by another device may be assisted.

The catheter device may be designed to be used in combination with a second device, e.g. an aspiration catheter and/or a microrobot. The distal anchor may be adapted to be delivered through a microrobot or another magnetofluidic device. Thus, the size of the anchor may be small which allows a selective anchoring on the distal surface of the blood clot. without contact with the distal edges of the blood clot and/or the artery wall. Moreover, the catheter device may not be adapted to move the blood clot without additional devices but may instead be adapted to initiate the displacement of the distal part of the blood clot when another device (e.g. a microrobot) is moving back after attachment to the blood clot.

The distal anchor, may be self-expandable, i.e. may be configured such as to expand without an external force acting on the anchor. Instead, the distal anchor may be held in a compressed configuration and released and/or the distal anchor comprise an activatable material which is activated by a stimulus or by removal of an activation element (e.g. a sheath).

The size of the distal anchor, in particular its diameter when expanded or before expansion, may be less than the length of a typical blood clot.

The catheter device reduces the risk of fragmentation of the thrombus, i.e. the breaking away of pieces of thrombus tissue.

Generally, for arterial ischemic stroke, a clot may be blocked in the internal carotid artery or middle cerebral artery mainly. Thus, the proximal diameter of the clot may typically be between 5 mm and 1 mm, preferably between 3 mm and 2 mm. The distal diameter may be between 5 mm and 1 mm, preferably between 3 mm and 2 mm. The proximal diameter and the distal diameter might be different.

Generally, devices known in the art have an outer diameter designed to exceed the diameter of the clot and may be in direct contact with the artery walls. Pulling of such devices may stretch the artery wall by friction forces. The stretching of the artery wall might induce damage to the vessel and to the peripheral vessels like bleeding. Thus, the catheter device may have, in particular an anchor portion in an activated state, a diameter between 0.5 mm and 2.5 mm in order to prevent a direct contact with the artery wall. Hence, the diameter of the activated anchor may be below the diameter of the artery.

The length of the catheter device may be between 100 cm and 300 cm, preferably between 170 cm and 240 cm. The catheter device may have one or several sections with different size, e.g. diameter and/or lengths.

The first shaft may have a substantially circular cross-section.

In an inactivated.state, i.e. when the distal anchor is not expanded, the distal anchor may have outer diameter between 200 µm and 2 mm, preferably between 250 um and 400 um. In the activated state, the distal anchor may have an outer diameter between 400 um and 5 mm, preferentially between 400 um and 1000 um.

The distal anchor may be made of hollow sections with an inner diameter between 100 um and 500 um, preferably between 170 µm and 250 um.

The catheter device, in particular the distal anchor, may comprise or consist of polymers, such as polyether-block-amide (PEBAX^{®}), and/or metals such as stainless steel or nitinol. The structure may be reinforced with coils or braided wires, e.g. to prevent kinking.

The catheter device, in particular the distal anchor and/or the first shaft, may be at least partially or fully radiopaque.

The distal portion of the shaft may be rigid enough to be pushed through the clot, and e.g. comprise a pushing wire. The catheter device may exert a pulling force of at least 100 mN during the retrieval.

Any part of the catheter device, in particular the first shaft and/or the distal anchor, may be coated. The coating may be a hydrophilic coating which may reduce friction during delivery, for example inside the medical device.

The catheter device may have a sensor to detect a contact with the clot. The sensor may be placed on the distal anchor and/or the distal portion of the first shaft. The sensor may be used to ensure that the distal part has moved through the clot. The sensor might provide information on the nature of the clot.

The distal anchor may be selected from a group comprising a balloon, at least one, preferably a plurality of, hinged arms, preferably two or four or six hinged arms, an umbrella, a wire structure, a retractive structure, and a net.

The catheter device may comprise a second shaft arranged concentrically around the first shaft. The second shaft may be configured to hold the distal anchor in a compressed configuration. A translational movement of the first shaft relative to the second shaft may cause expansion of the distal anchor.

The distal anchor may comprise an attachment element, for example a suction mechanism, to secure the thrombus to the distal anchor.

Thereby, an additional holding force may be provided on the thrombus and the force may be distributed more evenly on the thrombus surface. This may further reduce the risk of fragmentation of the thrombus.

The catheter device may comprise a radio-opaque element. Preferably, the radio-opaque element may be arranged on the first shaft and/or the distal anchor.

A plurality of radio-opaque elements may be arranged on the catheter device at one or several positions.

Radio-opaque elements allow a user to track the position and conformation of the device and/or the distal anchor via imaging techniques.

A radio-opaque element may comprise or consist of a radio-opaque material such as a barium compound, iodine, tantalum, platinium, Nd-Fe-B alloy. The radio-opaque element consist of a radio-opaque material or comprise such a compound embedded in a matrix, e.g.a polymer or a ceramic, to form the radio-opaque element. For example, a BaSO₄ compound may be mixed with polyethylene.

The radio-opaque element may have a thickness between 50 um and 500 um. The radio-opaque may be an element or a coating. The shape of the radio-opaque element may be a ring, a strip, and/or a sphere. The width of the radio-opaque element may be between 20 um and 500 um, preferably between 100 um and 200 µm. In one configuration, an element is radio-opaque by a coating on its surface or it is made with a radio-opaque mixture. The mixture may be made of a radio-opaque powder with a particle diameter between 5 nm and 5 um, preferably between 20 nm and 100 nm.

The distal anchor may comprise a first magnetic element.

The first magnetic element may assist the navigation and placement via an interaction with an external magnet and/or may be used to assist a pull-back motion, e.g. by interaction with a magnet placed proximally, e.g. on another medical device or, slidably arranged, on the catheter device.

The invention is further directed to a system for removing a thrombus from a vessel. The system comprises a catheter device as described herein above and a second medical device. The second medical device comprises a third shaft and a suction cup attached to the third shaft at a distal end of the third shaft. The third shaft comprises an inner channel which is in fluid communication with the suction cup. The suction cup is adapted to engage the thrombus and/or attach to the thrombus, from a proximal side, and the catheter device is at least partially deliverable through the second medical device, preferably through the third shaft.

The system therefore allows to attach to a thrombus from a proximal side, to provide a proximal pulling force, using the second medical device and additionally provide distal force via the catheter device. The removal of the thrombus is thereby more safe and fragmentation may be avoided as the force exerted on the thrombus is distributed more evenly and the internal stress on the thrombus is minimized.

Delivery of the catheter device may be done through the medical device which avoids the need for a separate delivery system. Once the distal anchor is behind the clot, the device may be activated to anchor the distal anchor on the distal surface of the clot. The state of the distal anchor may be controlled through a trigger system which may be located on the proximal side of the medical device. The catheter device may be moved through the lumen of the medical device and through the suction cup, or through a specific lumen and exiting before or in the suction cup.

A thrombus may be blocked in a straight artery, a tapered artery, a curved artery, or a bifurcation. Hence, in a tortuous vascular network, it might be challenging to reach the proximal surface with large catheters. Moreover, during this step, the navigation of large devices can lead to artery damages.

The suction cup may have an outer and inner size higher than other sections of the medical device. The suction cup, by its limited length, may be oriented and moved toward the proximal surface of the clot. This may reduce the damages to the arteries.

The catheter device may be deliverable through an opening of the suction cup or via an opening in the third shaft.

Alternatively, the second medical device may comprise a fourth shaft, preferably arranged in parallel to the third shaft, for delivery of the catheter device.

The second medical device may comprise a second magnetic element. The second magnetic element may, preferably, be formed as part of the suction cup and/or arranged on the distal end of the third shaft. The second magnetic element is adapted to interact with an external magnet for guiding the second medical device.

The third shaft may at least partially be formed as a flexible line.

The second medical device may, in particular, be adapted for magneto-fluidic navigation. Preferably, the third shaft has a proximal portion which is more rigid than a distal portion.

The flexible line may have a bending stiffness in the range of 1 mN•mm² to 2000 mN•mm², preferably between 50 and 400 mN•mm². The flexible line might be reinforced to limit its stretchability, e.g. during the clot retrieving. In some embodiments the flexible line may comprise at least one reinforcement. The reinforcement may extend along at least part of the flexible line. Alternatively, a plurality of reinforcement may be provided along at least part of the length of the flexible line. The reinforcement may be configured to prevent the flexible line of the device from deforming and/or kinking during navigation. The reinforcement may be configured to reinforce the flexible line at its position. For instance, the reinforcement may increase the stiffness of the flexible line at its position. This allows the flexible line to be flexible enough to be easily navigated through the vessel to the thrombus whilst also having the stiffness necessary to avoid twisting and kinking of the flexible line.

Optionally, a plurality of reinforcements may be provided along the flexible line. The plurality of reinforcements may be at least partly distanced from one another. The plurality of reinforcements may provide the flexible line with varying degrees of stiffness.

It is conceivable that at least one reinforcement may be any one or more of: a longitudinal reinforcement; a circumferential reinforcement; helical reinforcement; a lattice reinforcement. The reinforcement may be shaped as any one or more of: a ring; a coil; a ribbon; a grid; a lattice; a tube; a rod; ribbon.

The reinforcement may be embedded in the body of the flexible line. Additionally or alternatively, the reinforcement may be layered on the outer surface of the flexible line. Further additionally or alternatively, the reinforcement maybe provided as a layer on the inner surface of the flexible line.

In some embodiments, the reinforcement may be configured to allow visibility of the flexible line as it is navigated within the patient. To achieve this, at least a portion of the reinforcement may comprise a radiopaque material. Preferably, the reinforcement may comprise sections of radiopaque material. These sections of radiopaque material can optionally be spaced by sections of non-radiopaque material.

The invention is further directed to a system, preferably a system as described herein above, having a catheter device as described herein above and comprising a further medical device which is configured as an aspiration catheter.

The invention is further directed to a method of removing a thrombus form a patient's vessel. The method may be performed using a system as described herein above. The method comprises delivering a medical device which has a suction cup to a thrombus which is located in a patient's vessel. The medical device may be part of a system as described herein above. The suction cup is attached to a proximal side of the thrombus via suction which provides a suction force. A catheter device is delivered, preferably after attachment of the suction cup to the thrombus. Preferably, the catheter device is a catheter device as described herein above. The catheter device may at least partially be delivered through the medical device. The catheter device then penetrates the thrombus or passes by the side of the thrombus, from a proximal side to distal side. A distal anchor of the catheter device is deployed, preferably expanded, and attached to the distal side of the thrombus. The thrombus is retrieved by pulling on the medical device and/or the catheter device.

After attachment of the suction cup to the thrombus, but before attaching the distal anchor of the medical device to the thrombus, the medical device may be pulled backwards.

Thereby, slack may be reduced, in particular where the medical device is partially formed by a flexible line.

The distal anchor of the medical device may be activated, preferably expanded, before attaching to the thrombus.

The thrombus may be pulled, by pulling on the medical device and/or the catheter device into a retrieval chamber The retrieval chamber may be part of an aspiration catheter.

Preferably, no lytic agent is used in the method according to the invention. Use of a lytic agent may increase the risk of internal bleeding and/or of blood clot fragmentation.

Preferably, the distal anchor is only expanded behind a distal part of the thrombus. The size of the distal anchor may be smaller than the length of the treated blood clot. Therefore, a small and rigid compressed structure may be passed inside a controlling line and reduces the radial force applied by the compressed distal anchor on the delivery channel and the controlling line.

In the method according to the invention, the blood clot is only anchored at a proximal and a distal part. The distal anchor may be moved between the vascular wall and the clot to avoid a damage to the blood clot structure.

In general, the method according to the invention may include removing the thrombus without breaking it apart/fragmenting it/disintegrating it.

The distal anchor may be adapted to avoid direct contact with the vessel wall to avoid a damage of the endothelium during the retrieving and to avoid to stretching the artery.

In the following, the invention is described in detail with reference to the following figures, showing:
- Figs. 1a-1c:: schematically a use of a system according to the invention.
- Fig. 2:: schematically a system according to the invention.
- Figs. 3a-3c:: schematically a catheter device according to the invention.
- Figs. 4a-4b:: schematically a medical device according to the invention.
- Figs. 5a-5f:: schematically a method of using a system according to the invention.
- Figs. 6a-6b:: a catheter device according to a first embodiment of the invention.
- Figs. 7a-7b:: a catheter device according to a second embodiment of the invention.
- Figs. 8a-8d:: a catheter device according to a third embodiment of the invention.
- Figs. 9a-9b:: a catheter device according to a fourth embodiment of the invention.
- Figs. 10a-10b:: a catheter device according to a fifth embodiment of the invention.
- Figs. 11a-11b:: a catheter device according to a sixth embodiment of the invention.
- Figs. 12a-12b:: a catheter device according to a seventh embodiment of the invention.
- Fig. 13:: a system according to a first embodiment of the invention.
- Fig. 14:: a system according to a second embodiment of the invention.
- Fig. 15:: a system according to a third embodiment of the invention.
- Fig. 16:: a system according to a fourth embodiment of the invention.
- Fig. 17:: a system according to a fifth embodiment of the invention.
- Fig. 18:: a system for magnetic guidance.
- Figs. 19a-19c:: a method using an aspiration catheter.
- Fig. 20:: a system having an aspiration catheter.

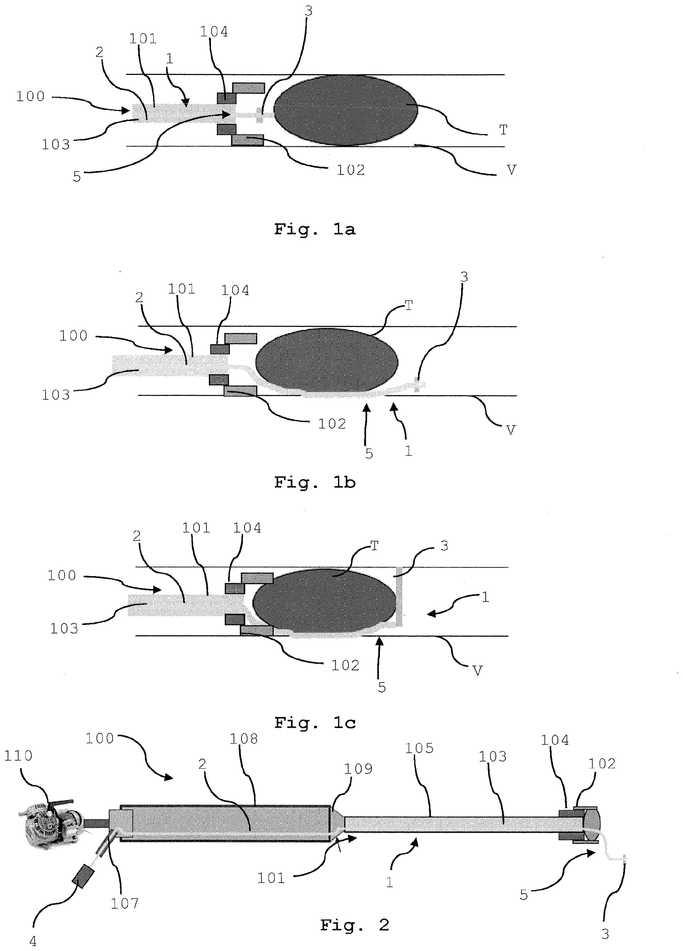

Figs. 1a-1c show, in an exemplary fashion, the use of a system according to the invention. The system is formed by a medical device 100 As shown in Fig. 1a, the system 100 and a catheter device 1. The medical device 100 comprises a shaft 101 with a suction cup 102 attached thereto at a distal end of it. In proximity to the suction cup 102, a magnetic element 104 is arranged with may be used to guide the medical device 100 to a desired treatment location, e.g. a thrombus T inside a vessel V. The shaft 101 is hollow and thereby forms a channel 103. Inside the channel, the catheter device 1 is arranged. The catheter device 1 has a distal anchor 3 arranged at a distal region 5 of shaft 2. The catheter device 1 is slidably arranged within the channel 103 of the medical device 100.

As shown in Fig. 1b, the catheter device 1 may be deployed out of the channel 103 and pass next to the thrombus T to its distal side. The distal portion 5 of shaft 2 passes between the thrombus T and the wall of vessel V. As shown here, the distal anchor 3 is inactivated and has a small diameter. By deploying the catheter device 1 through the medical device, the use of a separate delivery system may be avoided, thus reducing the space requirements at the treatment location.

Fig. 1c shows the deployed distal anchor 3 and with the suction cup 102 attached to the thrombus T. By simultaneous pulling of the catheter device 1 and the medical device 100, the thrombus T may be removed. It will be understood that the forces applied on the catheter device 1 and the medical device 100 may be varied depending on the circumstances.

The use of the catheter device 1 in combination with the medical device 100 or the suction device may be done according to the following steps:
- anchoring of the suction cup 102 on the proximal part of the thrombus T,
- monitoring the proximal anchoring;
- move backward the medical device 100;
- deliver the catheter device 1;
- position the distal anchor behind the thrombus T;
- activate the distal anchor 3;
- anchoring of the distal anchor 3 to the thrombus T;
- move backward the catheter device 1.

These steps may be done according to different combinations and thus different workflows. Preferably, the medical device is anchored to the thrombus and then the catheter device is delivered, positioned distally of the thrombus, activated and anchored. Then, the medical device and the catheter device may be moved together at the same velocity for the retrieving. In one workflow, the distal anchor is placed distally of the thrombus. Subsequently, the medical device is anchored, via the suction cup, and then the distal anchor of the catheter device is anchored. Alternatively, the distal anchor of the catheter device is anchored first and then the medical device is anchored.

For the retrieving, the medical device and the catheter device may be moved simultaneously or alternatingly. The devices may be moved at the same velocity. In one workflow, first the catheter device is moved to so as to move the distal portion. In one workflow, once the distal anchor is anchored and the suction cup is anchored, the medical device is moved forward so as to move the proximal part of the thrombus.

Fig. 2 shows a system according to the invention which comprises a catheter device 1 and a medical device 100 which is adapted for magnetofluidic navigation. The catheter device 1 may be a catheter device is a catheter device as shown in Figs. 1a-1c and is formed by a shaft 2 with a distal anchor 3 attached to its distal portion 5. The catheter device 1 is introduced into the medical device 100 through an access valve 107 and has, at its proximal end, an activation system 4. The medical device 100 is formed by a proximal portion 108 and a distal portion 105 connected by a connector 109, which together form a shaft 101. The distal portion 105 is formed as a flexible line. The shaft 101 is hollow and comprises an inner channel 103 through which the catheter device 1 is passed through. At a distal end of the medical device 100, a suction cup 102 is attached and in fluid communication with the inner channel 103. A magnetic element 104 is arranged concentrically around the distal end of the shaft 101 and behind the suction cup 102. A vacuum pump 110 is attached, via access valve 107, to the proximal portion 108 of the medical device 100 and configured to provide a vacuum to the inner channel 103, which in turn provides an aspiration force to the suction cup 102.

The anchor may be activated under a stimulus. The stimulus might be a chemical, physical, or mechanical stimulus. As an example, a mechanical stimulus may be the removal of a strain so that the distal anchor reaches an expanded configuration.

A chemical stimulus may be a prolongated contact with aqueous solution. For example, a dehydrated material might expand under contact with blood plasma. Additionally or alternatively, a specific activating solution may be injected to trigger the expansion.

A physical stimulus may refer, for example, to the activation by heat. For example, heat may be generated to a nitinol structure by applying a current.

The activation system may generate a physical, chemical or mechanical stimulus.

Heating may be done, for example, using joule heating, induction heating, or by injecting hot saline solution. The hot saline solution may be at a temperature above 37°C and preferably lower than 45°C.

Electrical wires may be used to deliver a current and heat up a part of the device, e.g. the distal anchor. The diameter of electrical wires may be between 10 µm and 200 µm, preferably between 40 um and 80 um. The electrical wires may have a flat cross-sectional shape and may be attached to the shaft and/or flexible line or may be formed on an outer wall of the shaft and/or flexible line, e.g. by a conductive coating. A width of the electrical wire may be between 10 um and 200 um. A thickness may be between 1 µm and 200 um.

Additionally or alternatively, the distal anchor may be compressed and may be navigated inside a sheath which may be pulled or pushed on demand to deploy or retract the distal anchor.

Additionally or alternatively, swelling polymers may be used to expand the distal anchor. For example, hydrogels (PVA, PVP, Polyacrylamide), porous hydrogels, superabsorbent polymers (Poly(acrylic acid (PAA), foams (PU) may be used as a matrix material in a biological attachment element to increase the contact surface with the thrombus after hydration by the blood.

The distal anchor may be coated with a thrombogenic agent such as thrombin. The coagulation reaction may improve the anchoring with the thrombus. To avoid unwanted thrombogenic reactions, e.g. during navigation and/or before treatment, the distal anchor may be covered. Preferably, the distal anchor is coated with a layer of polymer such as polyurethane, poly(lactic-co-glycolic acid (PLGA), and/or polydioxanone (PDO). The layer may have a thickness between 50 um to 300 um and may be biodegradable. The coating may be broken by flushing a solution, in particular a saline solution. Such a solution may be delivered via the flexible line.

The inner surface of the distal anchor may be configured to increase its temperature to heat the surface of the thrombus and thus creating cauterization. As a result, the thrombus may be attached more securely. The temperature may be increased up to 60°C, preferably to a temperature between 40 to 45°C, e.g. by heating a metallic surface through a Joule effect.

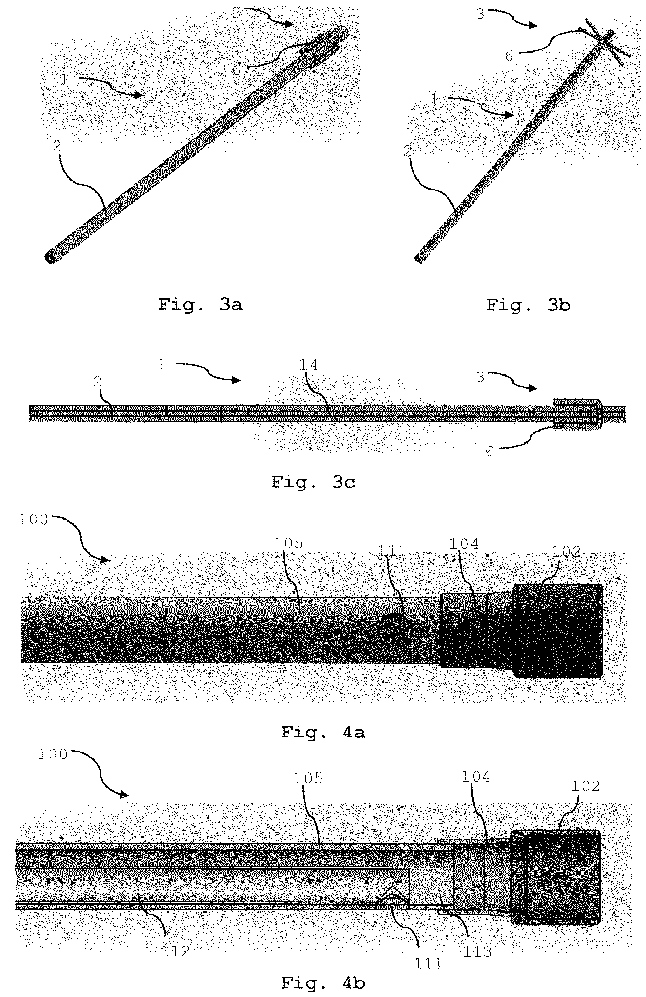

Fig. 3a shows an embodiment of a catheter device 1 having a shaft and six hinged arms 6 which are shown in an inactivated/compressed position parallel to shaft 2. The distal anchor 3, in the compressed configuration shown here, has a total diameter of approximately 0.5 mm. The diameter of each arm 6 is 0.1 mm, and each arm is made of a nitinol alloy. The length of each arm 6 is 1 mm.

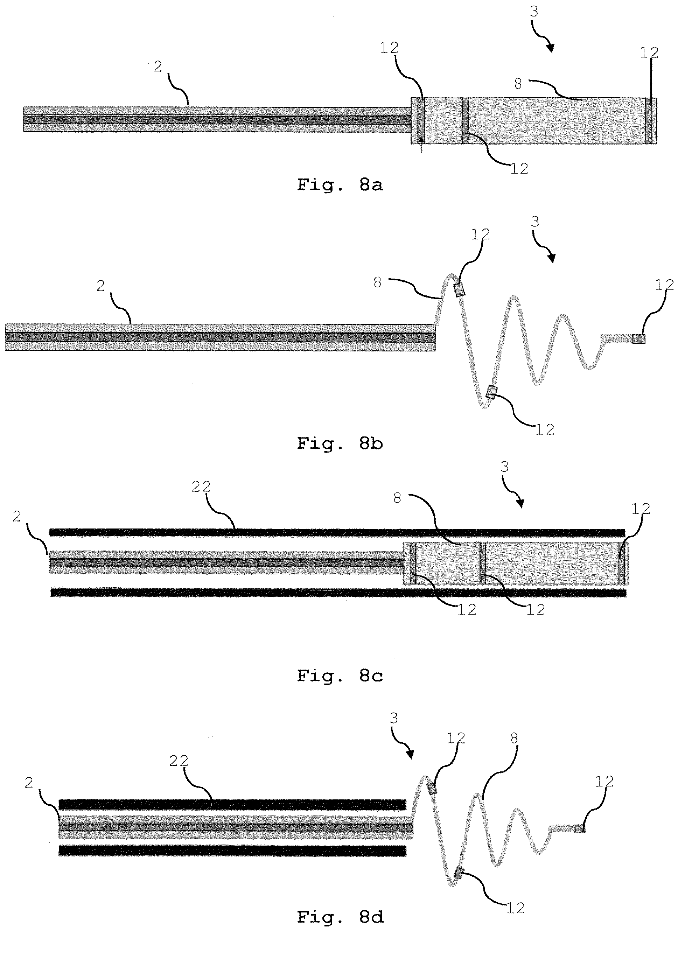

Fig. 3b shows the catheter device 1 of Fig. 3a in a deployed/expanded configuration. The distal anchor 3 may be deployed under a mechanical or physical effect. Here, the hinged arms 6 are triggered by a Joule effect induced by applying a current. Alternatively (see Figs. 8c/8d), the distal anchor may be in a bent configuration thanks to a shelf or a ribbon which is moved to allow the deployment.

Fig. 3c shows the catheter device 1 of Fig. 3a in a cross section. Here, the inner channel 14 is visible. The shaft 2 of the catheter device 1 has an outer diameter of 0.3 mm, and an inner channel 14 formed therein has an inner diameter of 0.1 mm. The shaft is made of stainless steel or nitinol.

Fig. 4a shows a medical device 100 with a suction cup 102, a flexible line 105, a magnetic part 104, and a valve 111. The valve is in fluid communication with, and closes, a separate delivery shaft (see Fig. 4b) for deployment of a catheter device (not shown). The medical device 1 is adapted to optimize the proximal anchoring to a thrombus. In the example, the flexible line 105 has an inner diameter of 1.2 mm and an outer diameter of 1.5 mm.

Fig. 4b shows a cross-section of the medical device 100 of Fig. 4a. The flexible line 105 has a delivery channel 112 for the a catheter device (not shown). Here, the magnetic element 104 and the suction cup 102 together form a cone-like structure with an inner diameter of 1.9 mm, an outer diameter of 2.15 mm, and a depth of 1.7 mm. The inner diameter of the delivery channel 112 is 0.75 mm and the outer diameter is 0.85 mm. In this example, the distal part of the delivery channel is occluded with a stopper 113. Via the stopper 113, the distal portion of a catheter device (see Figs. 1a-1c) may be bent when moved forward. To avoid a vacuum leak, a valve 111 is arranged distally on the flexible line 105 to allow the passage of a catheter device. A valve may also be present in the delivery channel 112. The valve may be made of silicone, polyurethane, and/or or rubber. The delivery channel 112 may be formed during the extrusion of the flexible line 105 or fixed to the flexible line 105, for example by gluing. By using a dedicated delivery channel 112, passing of a catheter device through the suction cup 102 may be avoided, which might increase the safety of the treatment due to possible impacts on the anchoring of the suction cup 102 on the proximal surface of the thrombus, e.g. the formation of a gap between the suction cup and the thrombus.

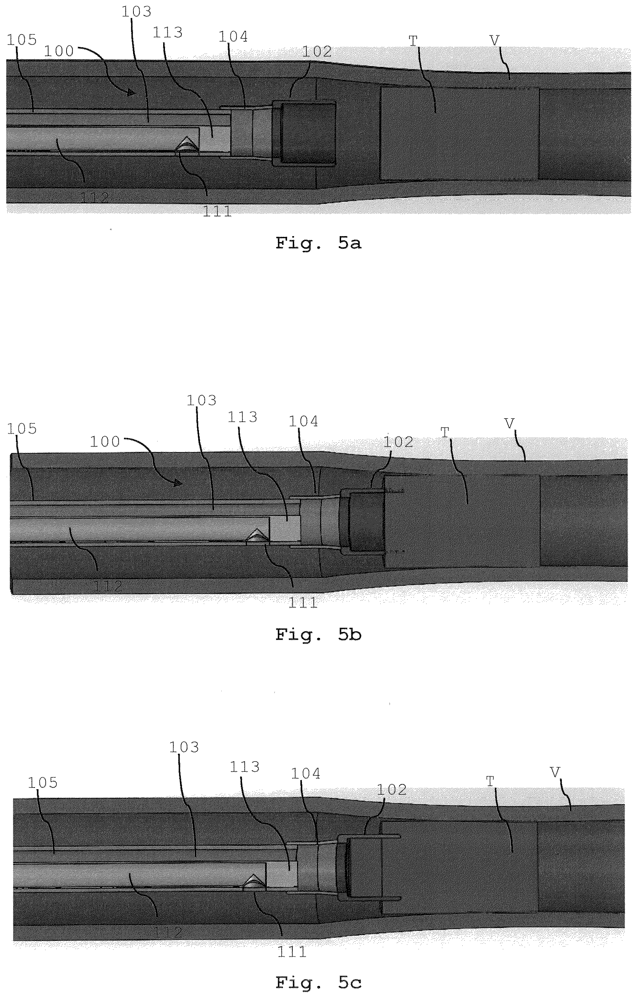

Figs. 5a-5f shows several anchoring steps for a medical device formed by a magnetic suction device, as shown in Figs. 4a and 4b, and a catheter device, in a blood vessel V having a proximal inner diameter of 3.6 mm and a distal inner diameter of 3 mm.

Fig. 5a shows the medical device 100 of Fig. 4a approaching a thrombus T in a vessel V.

In Fig. 5b, the suction cup 102 has attached to the thrombus T via a suction action provided via inner channel 103.

The thrombus T may partially be sucked into the suction cup 102, as shown in Fig. 5c.

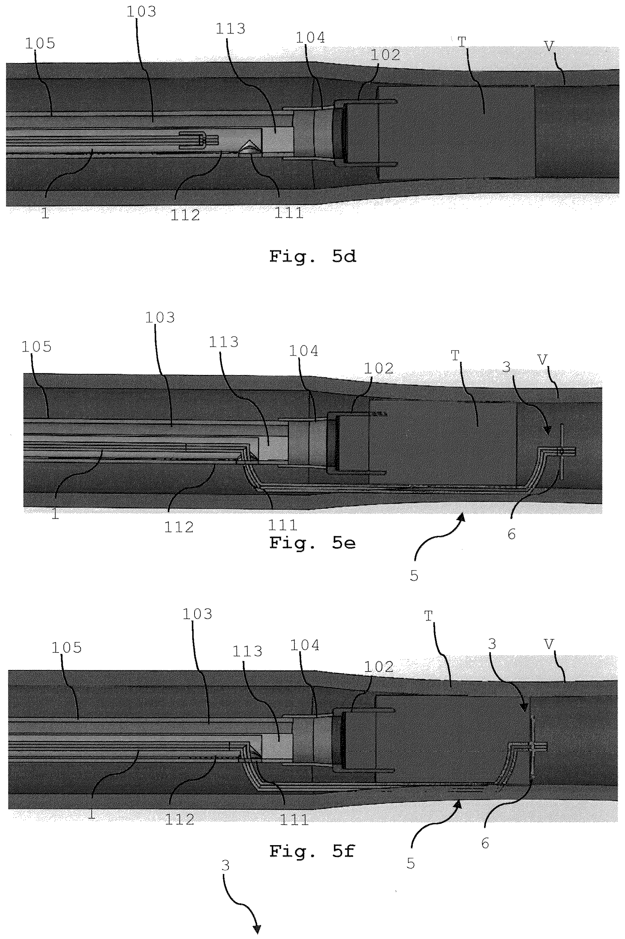

Through delivery channel 112, a catheter device 1 may be delivered (Fig. 5d). The catheter device 1 is a catheter device 1 as shown in Figs. 3a-3c.

As shown in Fig. 5e, the catheter device is deployed through valve opening 111 and passes by the thrombus T in between a thrombus wall and a vessel wall. The distal anchor 3, formed by hinged arms 6, is expanded once it reaches a position distal of the thrombus.

Fig. 5f shows how the distal anchor 3 is then pulled back to engage the thrombus T and pull it backwards. An additional pulling force may be exerted on the suction cup 102, via the flexible line 105, to remove the thrombus T from the patient's vessel V.

The magnetic suction medical device 100 is anchored to the clot to allow a better stability for the delivery of the distal anchor 3. The catheter device 1 is moved forward into the delivery system and moved on the other side of the thrombus T. Once in the distal area of the thrombus T, the distal anchor is activated. Then, the distal anchor 3 is moved backward up to the contact with the distal surface of the thrombus.

A clot may generally be blocked at a bifurcation, requiring a positioning ofthe medical device at the entrance of the occluded artery in a region of the bifurcation. Therefore, blood flow towardan open portion artery at the bifurcation. This blood flow may induce a drag force on the medical device away from the clot... Consequently, the suction head might be pushed to another blood vessel instead of the target artery.

Moreover, to reach the occluded artery, the medical device has to navigate in a tortuous vascular network. Once the distal part of the medical device has reached the occluded artery, the device on its length has several contact points on different arteries. These contact, points might move by applying radial force inside the medical device. Consequently, the distal part of the medical device might be moved backward. Thus the distal part of the medical device is now in the bifurcation and it may be pushed with guidance into the non-occluded artery.

The catheter device 1, in an inactivated state, has a diameter adapted to pass in the channel 112 of the medical device 100 and may have a diameter corresponding to 80% or less of a channel diameter, preferably corresponding to 60% to 40% of the channel diameter. Consequently, the catheter device 1 will not apply a radial and longitudinal force on the distal portion 105 during its delivery via the medical device 100.

The channel 112 is an optional feature. This channel 112 might be coated with an hydrophilic coating to improve the delivery of the catheter device 1. Moreover, this channel allows to deliver the catheter device 1 on the perimeter of the artery which is useful to pass the catheter device between the clot and the artery wall.

In one configuration, this channel might be outside of the catheter device, attached or connected to the outer surface 105.

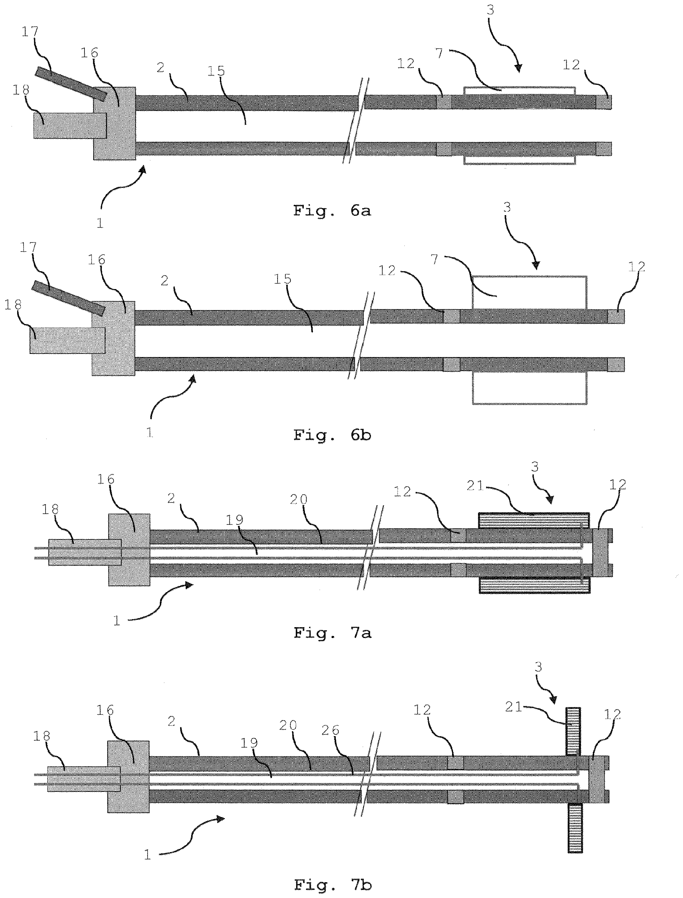

Fig. 6a shows an embodiment of a catheter device 1 having an inflatable distal anchor 3 in the form of a balloon 7. Furthermore, the catheter device 1 has radio-opaque markers 12 arranged both distally and proximally relative to the balloon 7. A channel 15 may be used to inflate the balloon 7. The balloon 7 may be inflated by injection of a liquid or a gas. Optionally, the catheter 1 has two channels, one each for inflating the balloon 7 and one for the handling, such as passing of a guidewire (not shown) which may assist in placing of the catheter device. Moreover, the hollow channel 15 allows the injection of solution such as contrast solution. The distal injection of contrast solution might help to identify the length of the thrombus and further distal occlusion. The catheter device 1 further has an access valve 16 with an injection port 17 for distal anchor inflation and an injection port 18 for a second hollow channel, when present. The balloon is made of a polymer, for example polyurethane or silicon. The balloon 7 may be reinforced with metallic wires, for example a nitinol wire, which may have a diameter between 30 µm and 100 um. Shaft 2 has an inner and outer diameter of 150 µm and 300 µm, and about 350 µm outer diameter including the uninflated balloon.

Fig. 6b shows the catheter device 1 of Fig. 6a with its balloon 7 inflated. When inflated, the balloon 7 has a diameter of 800 µm.

Fig. 7a shows a further embodiment of a catheter device 1 with a distal anchor in the form of an umbrella anchor 21. The umbrella anchor 21 comprises a frame which can change its longitudinal orientation to a radial orientation once activated (see Fig. 7b). The frame comprises several wires, rods or bars. The entire frame may be covered with a layer or a net. Here, activation wires 26 are present, which may be formed as electrical wires to generate heat and release of umbrella anchor 21. Umbrella anchor 21 may be made of an activable part (e.g. nitinol) and an inactivable part (e.g. stainless steel). The activable part may allow the orientation. The frame may also be activated under a chemical, physical or mechanical stimuli. Here, the catheter device 1 has an access valve 16 with a port 18 through which an activation wire 20 extends, through channel 19, and allows to activate the umbrella anchor 21. Radio-opaque elements 12, similar to the embodiment of Fig. 6a, are present here. The diameter of the inactivated umbrella anchor 21 is about 350 µm, whereas shaft 2 has an inner and outer diameter of 100 µm and 200 um, respectively.

The angle of the anchor 21, when activated and with respect to a main axis of the device 1, may be between 10° and 150°, preferably between 75° and 90°. The angle may be tuned according to the current value applied.

The anchor 21 might be coated to improve its adhesion with the clot. The coating might be made of an adhesive material such as protein-based (fibrin-based, collagen-based, gelatin-based, albumin-based) and/or polysaccharide-based adhesives (chitosan-based, alginate-based (e.g., SealG), and chondroitin-based). Moreover, the surface of the anchor may be coated with a thrombogenic agent by means of CVD, plasma spraying, or physical vapor deposition. Exemplary thrombogenic elements which may be employed are Von Wilerbrand factor (vWF), laminin, thrombospondin, vitronectin, fibrinogen or Thromboxane A2. These thrombogenic elements may trigger platelet adhesion and thus clot formation.

Fig. 7b shows the catheter device 1 of Fig. 7a with an expanded umbrella 21. The diameter of the activated umbrella anchor 21 structure is about 1.5 mm.

Fig. 8a shows a catheter device 1 with a retractive structure 8 (in the form of an activatable wire) which is configured to change from a straight configuration to a bent configuration. The retractive structure 8 may be formed of a shape memory material, such as a nitinol alloy. The retractive structure may be bent in one or two directions while being retracted in a third direction. Here, radio-opaque elements 12 are arranged at a proximal and a distal end of the retractive structure 12. The retractive structure is arranged on the shaft 2.

Fig. 8b shows the catheter device 1 of Fig. 8a, wherein the rectractive structure 8 has retracted, thereby expanding in a direction perpendicular to the direction of retraction (parallel to the shaft 2 in the present illustration).

Fig. 8c shows a catheter device 1 which is similar to the one shown in Fig. 8a. Here, the retractive structure 8 is pre-bent and held in a straight configuration by an outer frame 22. The outer frame 22 may be moved backwards, relative to retractive structure 8, to remove the stress applied on the distal anchor 8. The outer frame has an inner diameter between 0.5 mm and 1.2 mm.

Fig. 8d shows the embodiment of Fig. 8c wherein the outer frame 22 is moved proximally so as to release the retractive structure 8.

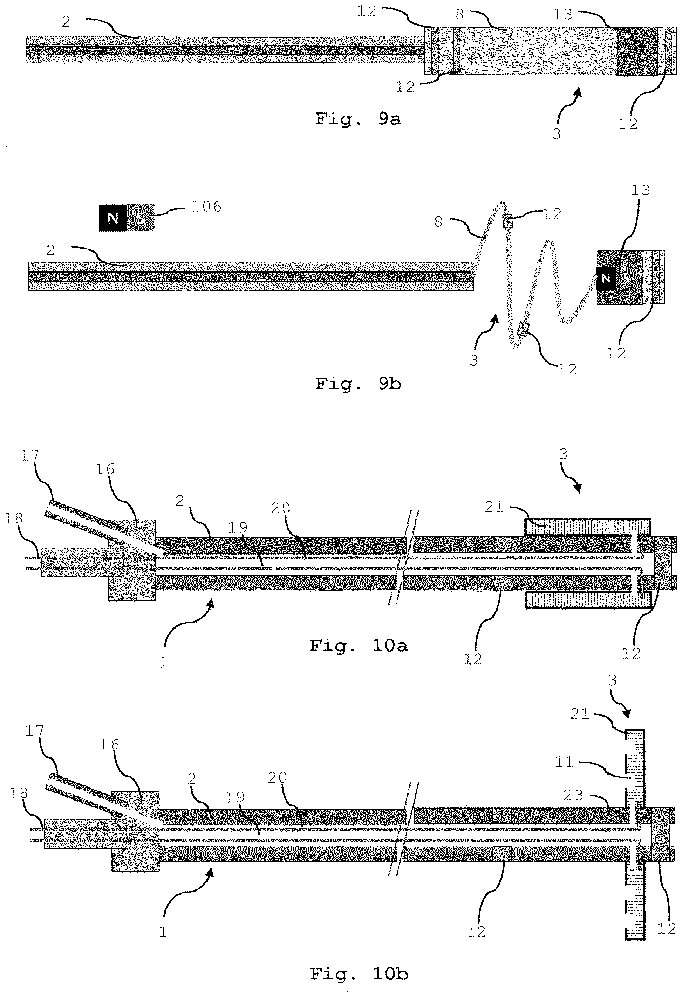

Fig. 9a shows an embodiment similar to the one shown in Figs. 8a-8d. Here, the retractive structure 8 has comprises a magnetic element 13. Under a magnetic field, the magnetic element is attracted the direction of the magnetic field. Before retraction, the diameter of retractable structure 8 is about 400 µm.

As shown in Fig. 9b, by interaction with an external magnet 106, the retractive structure 8 may be retracted. The diameter of the retracted retractable structure is about 1.1 mm.

The embodiment of Figs. 9a-9b is particularly suited to orient the retractive structure 8 in the same direction as the magnetic suction cup (not shown). Moreover, the distal anchor 3 may be attracted by the suction cup (not shown) leading to a better entrapment of the thrombus during the retrieving. The magnetic element 13 may be formed as an electromagnetic coil which is magnetized by applying a current.

The magnetic part 19 may be coated to prevent direct contact with a biological fluid and thus avoid corrosion. For example, coating materials against corrosion can be polymers (such as Parylene C), ceramics (such as silica based, zirconium based, TiO₂) or metals (gold, silver).

Fig. 10a shows a catheter device which is substantially similar to the embodiment of Figs. 7a-7b. Here, a port 17 is present for application of vacuum and in fluid communication with channel 19. The inner and outer diameter of shaft 2 are 200 um and 300 um, respectively, and approximately 450 µm including umbrella 21. When expanded, the umbrella 21 has a diameter of about 1.5 mm.

As shown in Fig. 10b, the umbrella anchor 21 may be expanded by activation of wire 20 in channel 19 (see also Figs. 7a and 7b). On a proximal side of the umbrella anchor 21, openings 11 are arranged which are in fluid communication with channel 19, via channels 23, allow application of a vacuum to the proximal surface of the umbrella anchor 21. Arms of umbrella anchor 21 may have inner diameters between 50 µm and 700 um. The umbrella anchor 21 is deployed under chemical, mechanical or physical stimuli. The vacuum may be applied once the distal anchor 3 is in contact with the distal part of the thrombus (not shown). The shaft of the catheter device 1 may be made of different channels to allow the injection of solution separately from the suction. A sensor (not shown) which is monitoring the vacuum may be used to detect and monitor the anchoring to the thrombus.

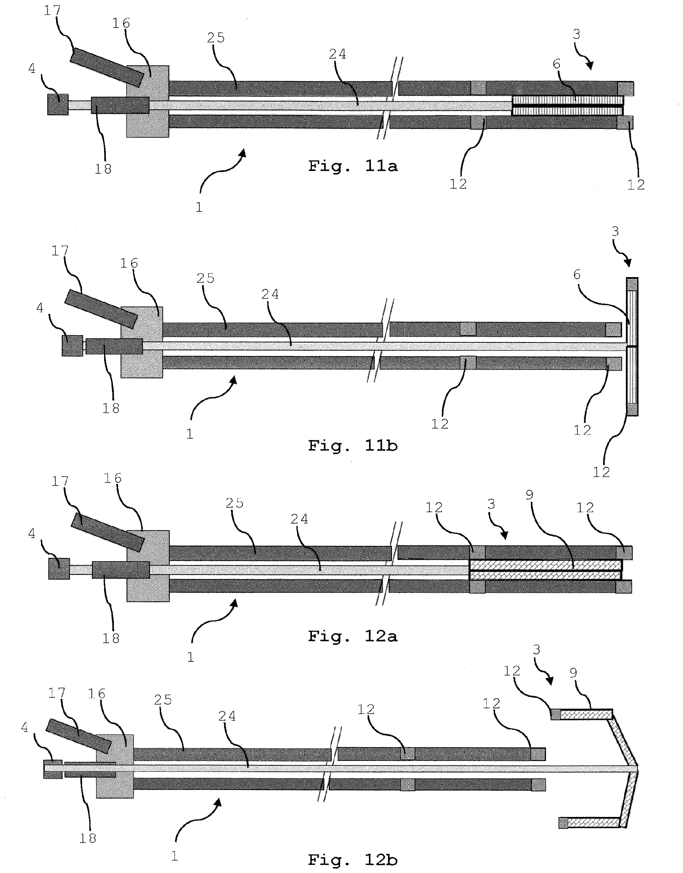

Fig. 11a shows an embodiment of a catheter device 1 wherein the distal anchor 3 is attached to an inner shaft 24. The distal anchor 3 is formed by hinged arms 6. The shaft 2 has an access valve 16 with ports 17, 18. Through port 18, the inner shaft 24 extends and is connected to activator 4. The inner and outer diameter of shaft 25 are 200 µm and 300 µm, respectively.

Therefore, the distal anchor 3 is mechanically compressed inside the shaft 25. The shaft may be formed by a microcatheter which may act as a delivery system for the distal anchor 3. Once the distal anchor 3 is pushed out of the shaft 25 by pushing on the activator 4, the distal anchor 3 expands due to the release of the compression by shaft 25. The shaft 25 formed as a microcatheter may be removed before removal of a thrombus (not shown). The diameter of distal anchor 3, when expanded, is 1.5 mm.

It is also conceivable that the distal anchor 3 is pushed out the delivery system in an inactivated state and then activated under a stimuli as described herein above.

To minimize the friction, the inner shaft 24 and/or the internal lumen of shaft 25 may be coated with an hydrophilic coating and/or a solution is injected during the delivery of the distal anchor 3. The shaft 25 may be coiled to avoid kinking and/or inappropriate stretching during the delivery of the distal anchor 3.

Fig. 12a shows a catheter device 1 which is substantially similar to the embodiments of Figs. 11 and 11b. Here, the distal anchor is formed by a net 9 instead of hinged arms 6. The distal part of the thrombus may be trapped inside the net structure 9. The distal anchor 3 is compressed in the shaft 25, for example formed by a micro-catheter. The distal anchor 3 is pushed out of shaft 25 by forward movement on the pushing element 4 and/or backward movement on the shaft 25. The distal anchor 3 may be released external of a distal end of shaft 25 and then moved back to the shaft 25. The inner and outer diameter of shaft 25 are 200 µm and 300 µm, respectively.

The anchoring with net 9 may be optimized by waiting at least several seconds, preferably at least 30 s, particularly preferably between 1 min and 2 min, before retrieval. Alternatively, the distal anchor 3 may be released in the vicinity of a distal side of the thrombus.

The net structure 9 may be made of polymer or metal, for example nitinol. The activated distal anchor 3 has a length between 1 mm and 20 mm, preferably between 2 min to 4 mm. The diameter of net structure 9, when expanded, is 1.5 mm. The net structure 9 may, optionally, be coated, e.g. with a polymer such as polyurethane.

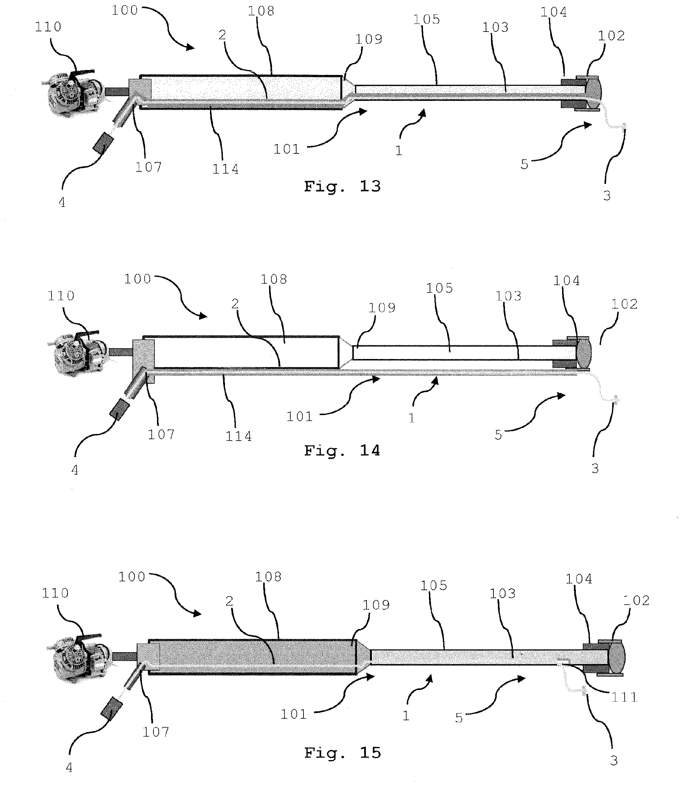

Fig. 13 shows a system according to the invention. The medical device 100 is similar to the one shown in Fig. 2, and identical features are not described repeatedly for clarity. Here, a dedicated channel 114 is arranged within shaft 101 and serves for introduction of the catheter device 1. Optionally, the channel 114 may have a valve at a distal part to limit vacuum propagation, which may, e.g., cause a leak in the vacuum system. The catheter device 1 may be any catheter device described herein. The channel 114 has an inner diameter of more than 100 µm, preferably between 250 µm and 600 um. The catheter device 1 exits the medical device 100 via the suction cup 102, which is in fluid communication with channel 114.

The suction cup 102 may be formed by two sections, e.g. which may be connected by a form fit or a snap fit, or may be formed integrally.

It will be understood that an aspiration catheter may be used instead of or in addition to the medical devices shown here.

Fig. 14 shows a further embodiment which is substantially similar to the system of Fig. 13. Here, the channel 114 for the catheter device 1 is arranged outside of shaft 101 and substantially parallel thereto.

The channel 114 may be made of polymers (PEBAX, polyurethane) and/or may be reinforced with a coil or braided wire (e.g. of nitinol or stainless steel).

Fig. 15 shows a further embodiment which is substantially similar to the systems of Figs. 13 ad 14. Here, no dedicated channel for the catheter device is present. Instead, catheter device 1 is introduced through port 17 and pushed through channel 103 formed by shaft 101 and exits through a separate opening 111 formed in the shaft 101 at a proximal position relative to the suction cup 102. The medical device 100 therefore has a distal exit formed by opening 111. In the lumen 103 of the suction medical device 100, a distal part of the port 111 is used to bend the catheter device toward outside. Opening 111 may be formed by a valve. The valve allows the passage of the distal anchor 3 and allows the sealing around the shaft 2 formed by pushing wire. The valve may be made of polymers, for example silicone. The height of the port is at least 300 um. The port might be made of a tubing with an elbow toward the valve.

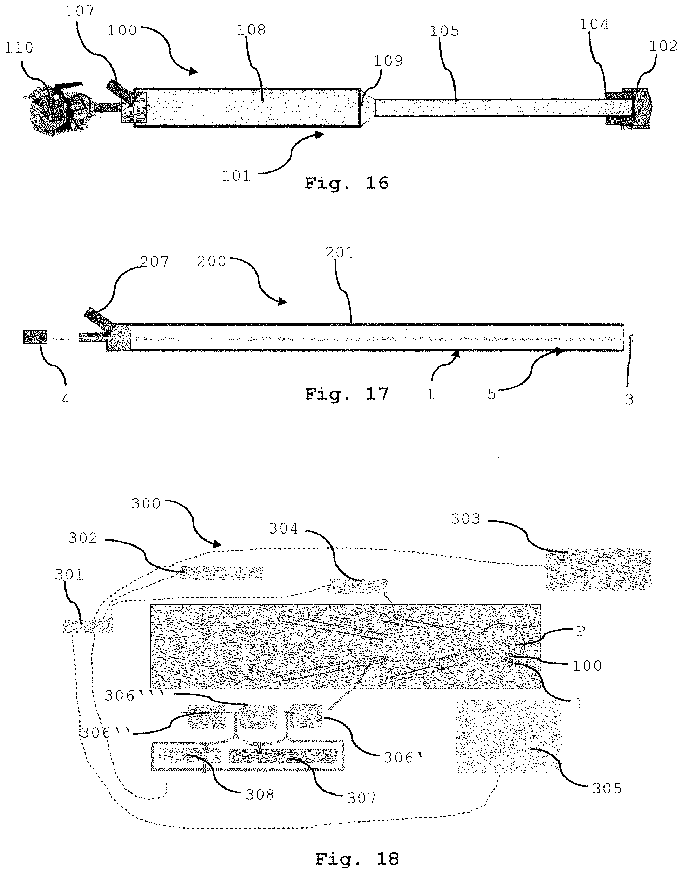

Fig. 16 shows the medical device 100 of Fig. 2 without a catheter device.

Fig. 17 shows a delivery device 200 having a shaft 201 and a port 207. The catheter device 1 is delivered via the delivery device 200 in proximity of a thrombus. Preferably, a suction medical device (see Fig. 16) is already anchored to the thrombus and the delivery device 200 is delivered preferably in parallel or via the medical device. A distal part of-the delivery device 200 may be magnetic to provide navigation. Additionally or alternatively, the delivery system may be advanced with a guidewire. The shaft 201 has an outer diameter of 350 µm and an inner diameter of 200 µm.

Fig. 18 shows a magnetic guidance system 300 for a medical device 100 and/or catheter device 1 as described herein above, and here shown when in operation in a patient P. The system 300 has a controlling system 301, a tracking system 302, a magnetic actuator 303, a patient sensor 304, an imaging system 305, three actuators 306', 306", 306‴, a pump 307 for solution injection, and a vacuum pump 308. The medical device 100 is actuated by the actuator 306' (and optionally by the actuator 306'''). Catheter device 1 is actuated by the actuator 306" and the magnetic actuator 303 and optionally by the actuator 306'''. The medical device 100 may be moved forward and backward with an actuator 306' controlled by the controlling system 301. Actuators 306'', 306‴ may also allow to generate the stimuli for the activation of the catheter device 1. A force sensor (not shown) might be used to detect the distal anchoring of the catheter device 1. Once the catheter device 1 is anchored, its backward displacement requires more force due to the presence of the thrombus. The force sensor may be placed proximally of the suction cup. The force sensor may also be connected to the magnetic navigation system.

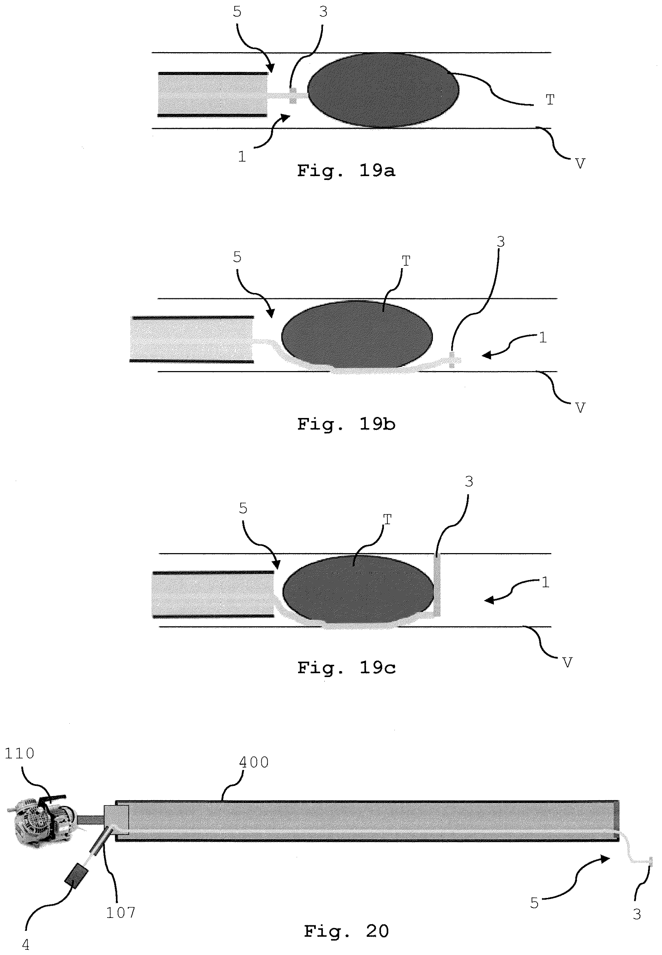

Figs. 19a-19c show the use of a system having a catheter device 1 with a distal anchor 3. The system is similar to the one shown in Figs. 1a-1c and identical or similar features are not described repeatedly for clarity. Here, instead of device 100, an aspiration catheter 400 with an internal chamber 401 is present. The thrombus T is aspirated by the aspiration catheter 400 and with the assistance of the catheter device 1.

Fig. 20 shows the system as used in Figs. 19a-19c in an overview. The system is similar to the one shown in Fig. 2, but is formed, here, by a catheter device 1 and an aspiration catheter 400. The aspiration cathter 400 has an internal chamber 401 for receiving a thrombus (not shown, see Figs. 19a-19c). Functionally similar or identical features as shown in Fig. 2 are not described repeatedly for clarity.

## Claims

1. A catheter device (1) for use in moving or removing, or assisting in moving or removing, a thrombus (T) within a vessel (V), comprising a first shaft (2), a distal anchor (3), and an activator (4),
wherein the distal anchor (3) is attached to a distal end portion (5) of the first shaft (2), and wherein the activator (4) is configured to expand the distal anchor(3),
**characterized in that** the distal portion (5) of the first shaft (2) is adapted engage the thrombus on a distal side,
preferably to penetrate the thrombus (T) and/or pass next to the thrombus (T), and further preferably extend from a first side of the thrombus (T) to a second side of the thrombus (T), such as to exert a force on the thrombus (T) from a distal side, via the distal anchor (3), when the first shaft (2) is pulled.

2. The catheter device according to claim 1, wherein the expandable distal anchor is selected from a balloon (7), one or more hinged arms (6), an umbrella (21), a wire structure (8), a retractive structure (8), and a net (9).

3. The catheter device according to any one of claims 1 or 2, further comprising a second shaft (10) arranged concentrically around the first shaft (2), wherein the second shaft (10) is configured to hold the distal anchor (3) in a compressed configuration, and wherein a translational movement of the first shaft (2) relative to the second shaft (10) causes expansion of the distal anchor (3).

4. The catheter device according to any one of the preceding claims, wherein the distal anchor (3) further comprises an attachment element, preferably a suction mechanism (11), to secure the thrombus (t) to the distal anchor (3).

5. The catheter device according to any one of the preceding claims, comprising a radioopaque element (12), preferably wherein the radioopaque element is arranged on the first shaft (2) and/or on the distal anchor (3).

6. The catheter device according to any one of the preceding claims, wherein the distal anchor (3) comprises a first magnetic element (13).

7. A system for removing a thrombus from a vessel, comprising a second medical device (100) and a catheter device (1) according to any one of the preceding claims,
the second medical device (100) comprising a third shaft (101) and a suction cup (102) attached to the third shaft (101) at a distal end thereof, wherein the third shaft (101) comprises an inner channel (103) which is in fluid communication with the suction cup (102),
the suction cup (102) being adapted to engage the thrombus and/or attach to the thrombus (T), from a proximal side,
wherein the catheter device (1) is at least partially deliverable through the second medical device (100), preferably the third shaft (101).

8. The system according to claim 7, wherein the catheter device (1) is deliverable via an opening of the suction cup (102), or via an opening (111) in the third shaft (101).

9. The system according to any one of claims 7 or 8, wherein the second medical device comprises a second magnetic element (104), preferably as part of the suction cup (102) and/or arranged on the distal end of the third shaft (101), wherein the second magnetic element (104) is adapted to interact with an external magnet (106, 303) for guiding the second medical device (100).

10. The system according to any one of claims 7 to 9,
wherein the third shaft (101) is at least partially formed as a flexible line (105).

11. A system for removing a thrombus (T) from a vessel (V), comprising a second medical device (100) and a catheter device (1) according to any one of the preceding claims, wherein the second medical device (100) is an aspiration catheter (400).

12. A method of removing a thrombus (T) from a patient's vessel, preferably using a system according any one of claims 7 to 10, comprising the steps of
- delivering a medical device (100) having a suction cup (102), preferably a medical device (100) which is part of a system according to any one of claims 7 to 10, to thrombus (T),
- attaching the suction cup (102) to the thrombus (T), via suction, on a proximal side of the thrombus (T),
- delivering a catheter device (1), preferably a catheter device (1) according to any one of claims 1 to 6, preferably through the medical device (100),
- penetrating the thrombus (T) and/or passing by the side of the thrombus (T), from the proximal side to a distal side, with the catheter device (1), and attaching a distal anchor (3) of the catheter device (1) to the distal side of the thrombus (T),
- retrieving the thrombus (T) by pulling on the medical device (100) and/or the catheter device (1).

13. The method according to claim 12, wherein the medical device (100) is pulled after attachment of the suction cup (102) to the thrombus (T) but before attaching the distal anchor the thrombus.

14. The method according to any one of claims 12 or 13,
wherein the distal anchor (3) is activated, preferably expanded, before attaching to the thrombus (T).

15. The method according to any one of claims 12 to 14,
wherein the thrombus (T) is pulled, by pulling on the medical device (100) and/or the catheter device (1), into a retrieval chamber (401), preferably a retrieval chamber of an aspiration catheter (400).
